(19) Europäisches Patentamt
European Patent Office
Office européen des brevets

(11) Veröffentlichungsnummer: **0 005 472**
**B1**

(12) # EUROPÄISCHE PATENTSCHRIFT

(45) Veröffentlichungstag der Patentschrift: **04.11.81**

(21) Anmeldenummer: **79101290.9**

(22) Anmeldetag: **30.04.79**

(51) Int. Cl.³: **C 07 C 69/65,** C 07 C 67/00 // C07C69/74, C07D303/32, C07D307/20

(54) **Verfahren zur Herstellung substituierter gamma-Halogencarbonsäureester.**

(30) Priorität: **13.05.78 DE 2821115**

(43) Veröffentlichungstag der Anmeldung: **28.11.79 Patentblatt 79/24**

(45) Bekanntmachung des Hinweises auf die Patenterteilung: **04.11.81 Patentblatt 81/44**

(84) Benannte Vertragsstaaten: **BE CH DE FR GB IT NL**

(56) Entgegenhaltungen:
**DE-A-2 544 150**
**DE-A-2 630 981**
**DE-A-2 731 483**
**US-A-3 992 422**
**US-A-4 021 459**
**CHEMICAL ABSTRACTS, Vol. 86, Nr. 19, 9. 05. 1977, Zusammenfassung Nr. 13941k, Columbus, Ohio, USA**

(73) Patentinhaber: **BAYER AG, Zentralbereich Patente, Marken und Lizenzen, D-5090 Leverkusen 1, Bayerwerk (DE)**

(72) Erfinder: **Mayer, Wolfram, Dr., Mozartstrasse 10, D-4150 Krefeld 1 (DE)**
Erfinder: **Heine, Hans-Georg, Dr., Am Heckerhof 14, D-4150 Krefeld 1 (DE)**

**EP 0 005 472 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung substituierter $\gamma$-Halogencarbonsäureester

Die vorliegende Erfindung betrifft ein verbessertes Verfahren zur Herstellung substituierter $\gamma$-Halogencarbonsäureester, ausgehend von entsprechenden Allylalkoholen und aliphatischen Orthoestern oder Ketenacetalen.

Es ist bereits bekannt geworden, daß bei der Umsetzung entsprechender Allylalkohole mit orthocarbonsäureestern oder Ketenacetalen $\gamma$-Halogencarbonsäureester entstehen (vgl. DE-OS 2 544 150). Bereits in DE-OS 2 544 150 wird auch erwähnt, daß bei dieser Reaktion verschiedene Nebenprodukte gebildet werden, wobei als Hauptprodukt jedoch der gewünschte $\gamma$-Halogencarbonsäureester entstehen soll. Aus DE-OS 2 731 483 geht hervor, daß bei der Umsetzung von Allylalkoholen mit orthocarbonsäureestern unter den bei DE-OS 2 544 150 angegebenen Reaktionsbedingungen substituierte $\gamma$-Lactonderivate entstehen.

Dies zeigt, daß der in DE-OS 2 544 150 angegebene Reaktionsverlauf uneinheitlich ist und den $\gamma$-Halogencarbonsäureester nicht in der für die Weiterumsetzung zum Cyclopropancarbonsäureester gewünschten Reinheit liefert.

Eine Nacharbeitung der aus DE-OS 2 544 150 und DE-OS 2 731 483 bekannten Reaktion ergab, daß bei der Umsetzung von Allylalkoholen mit Orthoestern oder Ketenacetalen ein Gemisch aus den entsprechenden $\gamma$-Halogencarbonsäureestern der Formel (I)

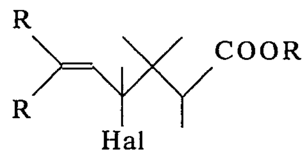

(I)

und den entsprechenden Ortholactonen der Formel (II)

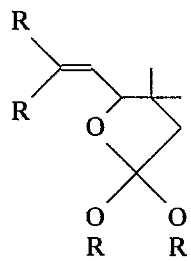

(II)

den entsprechenden $\gamma$-Lactonen der Formel (III)

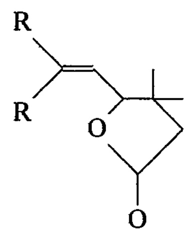

(III)

entsteht. Das Gemisch besteht zu einem großen Teil aus den $\gamma$-Halogencarbonsäureestern der Formel (I), als Nebenprodukt wird hauptsächlich das Ortholacton der Formel (II) gebildet, während das $\gamma$-Lacton der Formel (III) geringem Umfang entsteht.

Der Nachteil dieses Gemisches besteht jedoch darin, daß weder das Ortholacton noch das $\gamma$-Lacton auf technisch einfache Weise vom gewünschten $\gamma$-Halogencarbonsäureester getrennt werden kann. Beide Verunreinigungen gehen also in die Cyclisierung zum entsprechenden Cyclopropancarbonsäureester ein. Da auch hier eine technisch einfache Trennung nicht möglich ist, bleiben diese Verunreinigungen auch in den daraus hergestellten insektiziden Wirkstoffen enthalten. Dies führt dazu, daß die nach diesen Verfahren hergestellten insektiziden Cyclopropancarbonsäureester Verunreinigungen enthalten, die ihre Einsatzmöglichkeiten beeinträchtigen. Aus diesem Grund ist das in DE-OS 2 544 150 offenbarte Verfahren technisch von geringem Interesse.

Es war außerdem bekannt, daß $\gamma$-Lactone durch Behandlung mit Halogenierungsmitteln wie z. B. Thionylchlorid in die $\gamma$-Halogencarbonsäureester der Formel (I) überführt werden können (vgl. DE-OS 2 731 483 und DE-OS 2 630 981).

Es war jedoch nichts über das Verhalten der Ortholactone der Formel (II), die die hauptsächliche Verunreinigung bei dieser Reaktion darstellen, bekannt.

Es wurde nun ein Verfahren zur Herstellung von γ-Halogencarbonsäureestern der Formel (IV) gefunden

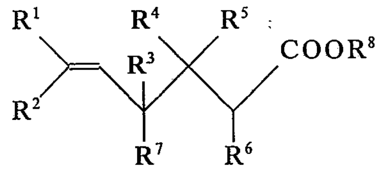

(IV)

in welcher

| | |
|---|---|
| $R^1$ und $R^2$ | gleich oder verschieden sind und für Wasserstoff oder Halogen stehen, |
| $R^3 - R^6$ | gleich oder verschieden sind und für Wasserstoff oder gegebenenfalls substituiertes Alkyl stehen, |
| $R^7$ | für Halogen steht, |
| $R^8$ | für einen gegebenenfalls substituierten Kohlenwasserstoffrest steht, |

durch Umsetzung einer Verbindung der allgemeinen Formel (V)

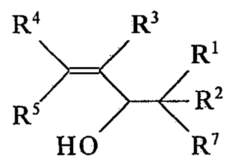

(V)

in welcher

| | |
|---|---|
| $R^1 - R^5$ | die oben angegebene Bedeutung haben und |
| $R^7$ | für Halogen steht, |

mit einem Orthoester der allgemeinen Formel (VI)

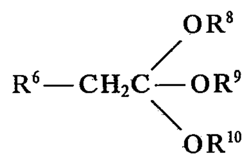

(VI)

in welcher

| | |
|---|---|
| $R^6$ | die oben angegebene Bedeutung hat und |
| $R^8$, $R^9$ und $R^{10}$ | gleich oder verschieden sind und für einen gegebenenfalls substituierten Kohlenwasserstoffrest stehen, |

oder mit einem Ketenacetal der allgemeinen Formel (VII)

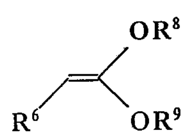

(VII)

in welcher

| | |
|---|---|
| $R^6$, $R^8$, $R^9$ | die oben angegebene Bedeutung haben |

gegebenenfalls in einem organischen Lösungsmittel und gegebenenfalls in Gegenwart eines sauren Katalysators, dadurch gekennzeichnet, daß man das bei dieser Umsetzung erhaltene Gemisch mit maximal 1 Moläquivalent eines Halogenierungsmittels behandelt.

Geeignete Produktgemische sind z. B. solche aus den Umsetzungen eines der folgenden Alkenole gemäß Formel (V)

1,1,1-Trichlor-4-methyl-pent-3-en-2-ol, 1,1,1-Tribrom-4-methyl-pent-3-en-2-ol, 1-Chlor-1,1-dibrom-1-methyl-pent-3-en-2-ol, 1-Brom-1, 1-dichlor-4-methyl-pent-3-en-2-ol, 1,1-Dibrom-4-methyl-pent-3-en-2-ol, 1,1-Dichlor-4-methyl-pent-3-en-2-ol, 1,1,1-Trichlor-4-methyl-hept-3-en-2-ol,

1,1,1-Tribrom-4-methyl-hept-3-en-2-ol, 1,1-Dichlor-4-methyl-hept-3-en-2-ol, 1,1-Dibrom-1-methyl-hept-3-en-2-ol, 1,1,1-Trichlor-4,6,6-trimethyl-hept-3-en-2-ol, 1,1,1-Tribrom-4,6,6-trimethyl-hept-3-en-3-en-2-ol, 1,1,1-Trichlor-4-äthyl-hex-3-en-2-ol, 1,1,1-Tribrom-4-äthyl-hex-3-en-2-ol, 1,1,1-Trichlor-4-methyl-hex-3-en-2-ol, 1,1,1-Tribrom-4-methyl-hex-3-en-2-ol, 1,1,1-Trichlor-hept-3-en-2-ol und 1,1,1-Tribrom-hept-3-en-2-ol.

Mit einem der folgenden Orthoestern gemäß Formel (VI)

1,1,1-Trimethoxyäthan, 1,1,1-Triäthoxyäthan, 1,1,1-Tricyclohexyloxyäthan, 1,1,1-Tri-n-butyloxyäthan, 1,1,1-Triäthoxypropan, 1,1,1-Triäthoxybutan, 1,1,1-Triäthoxypentan, 3-Methyl-1,1,1-triäthoxybutan, 3,7-Dimethyl-1,1,1-triäthoxyoctan, 2-Phenyl-1,1,1-triäthoxyäthan, 2-(o-Methylphenyl)-1,1,1-triäthoxyäthan, 2-(m-Methylphenyl)-1,1,1-triäthoxyäthan, 2-Cyclohexyl-1,1,1-trimethoxyäthan, 1,1-Dimethoxy-1-cyclohexyloxyäthan, 1,1-Dimethoxy-1-pentoxyäthan, 3-Methyl-1,1,1-triäthoxybuten, 1,1,1-Triäthoxy-6-heptin, 1-Benzyloxy-1,1-diäthoxyäthan, 1,1-Diäthoxy-1-geranyloxyäthan, 1,1,1-Tribenzyloxyäthan, 1,1,1-Trioctyloxyäthan, 1,1-Diäthoxy-1-octyloxyäthan, 1,1-Diäthoxy-1-prenyloxyäthan, 1,1-Diäthoxy-1-allethronyloxyäthan, 1,1-Diäthoxy-1-pyrethronyloxyäthan, 1,1-Diäthoxy-1-(3-phenoxybenzyloxy)-äthan, 1,1-Diäthoxy-1-(5-benzyl-3-furylmethyloxy)-äthan, 1,1-Diäthoxy-1-(tetrahydrophthalimidomethyloxy)-äthan sowie 1,1-Diäthoxy-1-(3-benzylpropargyloxy)-äthan.

Gegebenenfalls unter Zusatz von sauren Katalysatoren wie

Essigsäure, Propionsäure, Buttersäure, Isobuttersäure, Cyclohexylcarbonsäure, Valeriansäure, Malonsäure, Bernsteinsäure oder Adipinsäure, aromatische Carbonsäure, wie Benzoesäure und m-Chlorbenzoesäure, Phenole wie Phenol, o-Nitrophenol, m-Nitrophenol, p-Nitrophenol, o-Kresol, m-Kresol, p-Kresol, o-Xylenol, p-Xylenol, 2,6-Dimethylphenol, 2,6-Di-tert.-Butylphenol, 2,4,6-tri-sek.-Butylphenol, 2,4,6-tri-tert.-Butylphenol, 4-Methyl-2,6-di-tert.-Butylphenol, 4-Methyl-3,5-di-tert.-butylphenol, Hydrochinon, 2,5-di-tert.-Butylhydrochinon, $\alpha$-Napthol und $\beta$-Naphthol, Sulfonsäuren, wie Benzolsulfonsäure und p-Toluolsulfonsäure, Mineralsäuren wie Chlorwasserstoffsäure, Schwefelsäure, Phosphorsäure oder Borsäure, sowie Lewis-Säuren, wie Aluminiumchlorid, Zinkchlorid, Eisen(III)-chlorid oder Bortrifluorid.

Die Verwendung einer Fettsäure mit 2 bis 6 Kohlenstoffatomen, eines Phenols oder einer aromatischen Carbonsäure wird bevorzugt. Der saure Katalysator wird in einer Menge von 0,001 bis 20 Gew.-% und vorzugsweise in einer Menge von 0,01 bis 15 Gew.-%, bezogen auf das -1-Halogen-alk-3-en-2-ol der Formel (I), eingesetzt.

Geeignete Halogenierungsmittel sind z. B. Chlorwasserstoff, Thionylchlorid, Bromwasserstoff, Phosphortrichlorid, Thionylbromid, Phosphortribromid.

Geeignete Lösungsmittel können sein z. B. Alkohole wie Äthanol, Methanol, tert.-Butanol, n-Butanol; aromatische oder halogenierte Kohlenwasserstoffe wie Benzol, Toluol, Chlorbenzol, Chloroform, Tetrachloräthan.

Zur Durchführung des erfindungsgemäßen Verfahrens wird ein geeignet substituierter Allylalkohol der allgemeinen Formel (V) gegebenenfalls in einem aprotischen Lösungsmittel mit einem Überschuß (2—10 Moläquivalente) eines Orthoesters bzw. Ketenacetals der allgemeinen Formel (VI) oder (VII) bei Normaldruck und bei einer Temperatur von 120—170°, vorzugsweise bei einer Temperatur von 120 bis zur Siedetemperatur des verwendeten Orthoesters, in an sich aus der Lietratur bekannten Weise umgesetzt, wobei der sich bildende Alkohol kontinuierlich aus dem Reaktionsgemisch abdestilliert wird. Vorzugsweise wird so verfahren, daß der Umsatz des verwendeten subst. Allylalkohols durch GC-Analyse kontrolliert wird und hiernach Orthoester sowie als Katalysator eingesetzte Säure im Verlauf der Reaktion nachdosiert werden. Im allgemeinen ist die Reaktion nach 24 h so weit fortgeschritten, daß eine weitere Umsetzung des subst. Allylalkohols nicht lohnenswert ist. Zur Aufarbeitung kann das Reaktionsgemisch auf verschiedene Weise behandelt werden. Wurde eine starke Säure, wie z. B. p-Toluolsulfonsäure, verwendet, so kann das Reaktionsgemisch in eine verdünnte wäßrige Bicarbonatlösung gegeben werden. Durch Extrahieren mit einem organischen Lösungsmittel, wie z. B. Chloroform oder Toluol, werden die gewünschten organ. Verbindungen erhalten. Oftmals kann das Reaktionsgemisch direkt einer fraktionierenden Destillation unterworfen werden, wobei ein Gemisch von Reaktionsprodukten resultiert, in dem als Hauptprodukt eine Verbindung der allgemeinen Formel (IV) neben einem $\gamma$-Lacton und seinem Ortho-Derivat enthalten ist.

Ein solches Gemisch wird gegebenenfalls in einem geeigneten Verdünnungsmittel mit einem Halogenierungsmittel, dessen Menge 0,3 bis 1 Moläquivalente, bevorzugt 0,8 bis 1 Moläquivalente maximal also 1 Moläquivalent beträgt, bei Temperaturen zwischen 0 und 100°, vorzugsweise 20—80°, behandelt. Oftmals tritt hierbei eine positive Wärmetönung auf. Wird ein Verdünnungsmittel verwendet, so kann dieses Verdünnungsmittel gleichzeitig Reaktionspartner für die Umwandlung der Lactone sein, d. h. das Verdünnungsmittel kann ein Alkohol sein.

Zur Isolierung des Reaktionsprodukts kann fraktionierend destilliert werden. In vielen Fällen wird auf die Isolierung verzichtet und das aus der Umsetzung mit einem Halogenierungsmittel resultierende Reaktionsprodukt der allgemeinen Formel (IV) direkt in die Cyclisierung mit einer Base zu einem Cyclopropancarbonsäureester eingesetzt. Diese Cyclisierung ist an sich aus der Literatur bekannt und kann in verschiedenen Varianten durchgeführt werden (s. z. B. DE-OS 2 544 150).

Die folgenden Beispiele belegen das erfindungsgemäße Verfahren.

### Beispiel 1

### (Vergleichsbeispiel)

30,6 g 1,1,1-Trichlor-4-methyl-pent-3-en-2-ol werden mit 48,6 g 1,1,1-Triäthoxyäthan in Gegenwart von 0,3 g Isobuttersäure gemäß DE-OS 2 544 150 (Beispiel 1) umgesetzt. Fraktionierende Destillation liefert 30,0 g eines gelblichen Öls vom $Sdp._{0,2}$ 78—86°. Analyse des Destillats zeigt, daß außer dem Hauptprodukt 3,3-Dimethyl-4,6,6-trichlor-hex-5-en-säureäthylester folgende Nebenprodukte enthalten sind:

1) 3,3-Dimethyl-6,6,6-trichlor-hex-4-en-säureäthylester (75%)
2) 2-Oxo-4,4-dimethyl-5-($\beta$,$\beta$-dichlorvinyl)-tetrahydrofuran ($\leq$ 3%)
3) 2,2-Diäthoxy-4,4-dimethyl-5-($\beta$,$\beta$-dichlorvinyl)-tetrahydrofuran (22—25%)

27,0 g dieses Reaktionsgemisches werden in 200 ml trockenem Äthanol, in dem zuvor 4,0 g Natrium gelöst wurden, gemäß DE-OS 2 544 150 (Beispiel 7) cyclisiert. Aufarbeitung, wie beschrieben, liefert 12,0 g eines fast farblosen Öls vom $Sdp._{0,4}$ 80—95°. Laut NMR-Spektrum ($CDCl_3$) sind hierin außer dem erwarteten Permethrinsäureäthylester (Verhältnis der cis/trans-Isomeren 16 : 84) die obengenannten Verunreinigungen enthalten.

### Beispiel 2

#### a) Ausgangsgemisch

Die Lösung von 500,0 g 1,1,1-Trichlor-4-methyl-pent-3-en-2-ol und 20,0 g Isobuttersäure in 1200,0 g 1,1,1-Triäthoxyäthan wird innerhalb von 3,5 h auf 145° (Innentemperatur) erhitzt. Der gebildete Äthylalkohol wird hierbei kontinuierlich über eine Vigreux-Kolonne abdestilliert, während der Umsatz gaschromatographisch verfolgt wird. Nach 3,5, 13,5 und 21 h werden jeweils 10,0 g Isobuttersäure zugegeben. Nach 20 h werden außerdem 500,0 g 1,1,1-Triäthoxyäthan zugesetzt. (Verlust durch abdestillierenden Äthylalkohol und Veresterung der Isobuttersäure.) Nach 26 h wird die gelbbraune Lösung in 5,0 l 1% wäßrige Natriumbicarbonat-Lösung gegossen, die darauf mit Chloroform extrahiert wird. Trocknen der organischen Extrakte über wasserfreiem Natriumsulfat und fraktionierendes Destillieren liefern neben u. a. nicht umgesetztem 1,1,1-Triäthoxyäthan und Fraktionen, die Ausgangs- und Reaktionsprodukte enthalten, 410,0 g (60%) eines 75 : 25 Gemischs von 3,3-Dimethyl-4,6,6-trichloro-hex-5-ensäureethylester und 2,2-Diäthoxy-4,4-dimethyl-5-($\beta$,$\beta$-dichlorvinyl)-tetrahydrofuran. Der Anteil von 2-Oxo-4,4-dimethyl-$\beta$,$\beta$-dichlorvinyl-tetrahydrofuran liegt unter 3%.

2,2-Diäthoxy-4,4-dimethyl-5-($\beta$,$\beta$-dichlorvinyl)-tetrahydrofuran:

NMR ($CDCl_3$): $\delta$ = 1,05 s (3H, 1,13 s (3H), 1,20 f (6 H, J = 6 Hz), 1,96 s (2 H), 3,56 q (3 H, J = 7 Hz), 3,60 q (2 H), 4,55 d (1 H, J = 8,5 Hz) und 5,90 d (1 H)

IR ($CDCl_4$): keine Absorption im Bereich von 1690—1820 $cm^{-1}$.

#### b) erfindungsgemäße Behandlung

23,8 g des gemäß Beispiel 2 erhaltenen Destillats werden in 150 ml Benzol mit 11,9 g Thionylchlorid so lange auf 70° erhitzt, bis das Ortholacton umgesetzt ist (4—8 h). Anschließend werden 100 ml Äthylalkohol zugegeben. Nach 8 h Rückfluß wird fraktionierend destilliert. Man erhält 23,7 (99%) einheitlichen 3,3-Dimethyl-4,6,6-trichlor-hex-5-en-säureäthylester von $Sdp._{0,1}$ 69°.

#### c) Cyclisierung

Zu der Lösung von 34 g Natrium in 2000 ml Äthylalkohol werden bei 20° 326,0 g 3,3-Dimethyl-4,6,6-trichlor-hex-5-en-säureäthylester, nach Beispiel 3 bereitet, in 1000 ml Äthylalkohol zugetropft. Nach Rühren über Nacht (16 h) wird mit einer Lösung von Chlorwasserstoff in Äthylalkohol neutralisiert und

anschließend in Vak. der größte Teil des Lösungsmittels abdestilliert. Den flüssigen Rückstand versetzt man mit Wasser, extrahiert organische Verbindungen mit Toluol, wäscht einmal mit Wasser, trocknet über Natriumsulfat und dampft ein. Fraktionierende Destillation liefert neben 27,2 g Vorlauf 228,0 g (80%) einheitlichen 2,2-Dimethyl-3-($\beta$,$\beta$-dichlorvinyl)-cyclopropancarbonsäureäthylester vom Sdp.$_{0,25}$ 73°, $n_D^{20}$ 1,4867.

**Patentanspruch**

Verfahren zur Herstellung von $\gamma$-Halogencarbonsäureestern der Formel (IV)

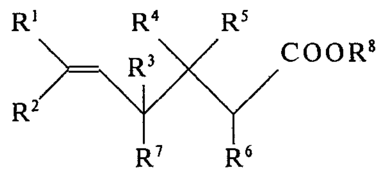

(IV)

in welcher

| | |
|---|---|
| $R^1$ und $R^2$ | gleich oder verschieden sind und für Wasserstoff oder Halogen stehen, |
| $R^3$ – $R^6$ | gleich oder verschieden sind und für Wasserstoff oder gegebenenfalls substituiertes Alkyl stehen, |
| $R^7$ | für Halogen steht, |
| $R^8$ | für einen gegebenenfalls substituierten Kohlenwasserstoffrest steht, |

durch Umsetzung einer Verbindung der allgemeinen Formel (V)

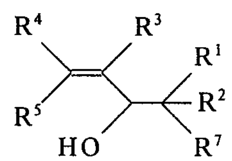

(V)

in welcher

| | |
|---|---|
| $R^1$ – $R^5$ | die oben angegebene Bedeutung haben und |
| $R^7$ | für Halogen steht, |

mit einem Orthoester der allgemeinen Formel (VI)

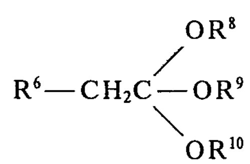

(VI)

in welcher

| | |
|---|---|
| $R^6$ | die oben angegebene Bedeutung hat und |
| $R^8$, $R^9$ und $R^{10}$ | gleich oder verschieden sind und für einen gegebenenfalls substituierten Kohlenwasserstoffrest stehen, |

oder mit einem Ketenacetal der allgemeinen Formel (VII)

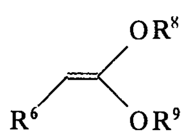

(VII)

in welcher

| | |
|---|---|
| $R^6$, $R^8$, $R^9$ | die oben angegebene Bedeutung haben |

gegebenenfalls in einem organischen Lösungsmittel und gegebenenfalls in Gegenwart eines sauren

Katalysators, dadurch gekennzeichnet, daß man das bei dieser Umsetzung erhaltene Gemisch mit maximal 1 Moläquivalent eines Halogenierungsmittels und eines Alkohols behandelt.

**Claim**

Process for the preparation of γ-halogenocarboxylic acid esters of the formula (IV)

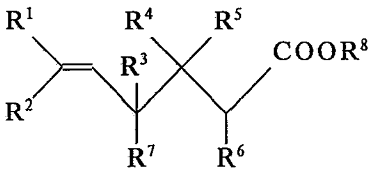

(IV)

in which

| | |
|---|---|
| $R^1$ and $R^2$ | are identical or different and represent hydrogen or halogen, |
| $R^3$ — $R^6$ | are identical or different and represent hydrogen or optionally substituted alkyl, |
| $R^7$ | represents halogen and |
| $R^8$ | represents an optionally substituted hydrocarbon radical, |

by reacting a compound of the general formula (V)

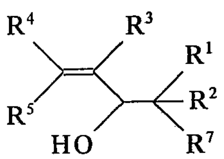

(V)

in which

| | |
|---|---|
| $R^1$ — $R^5$ | have the meaning indicated above and |
| $R^7$ | represents halogen, |

with an orthoester of the general formula (VI)

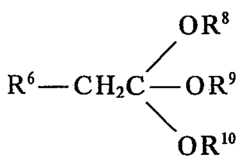

(VI)

in which

| | |
|---|---|
| $R^6$ | has the meaning indicated above and |
| $R^8$, $R^9$ and $R^{10}$ | are identical or different und represent an optionally substituted hydrocarbon radical, |

or with a ketene acetal of the general formula (VII)

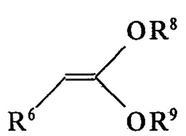

(VII)

in which

| | |
|---|---|
| $R^6$, $R^8$ and $R^9$ | have the meaning indicated above, if appropriate in an organic solvent and if appropriate in the presence of an acid catalyst, characterised in that the mixture obtained in this reaction is treated with at most 1 molar equivalent of a halogenating agent and an alcohol. |

**Revendication**

Procédé de préparation d'esters d'acides γ-halogénocarboxyliques de formule (IV):

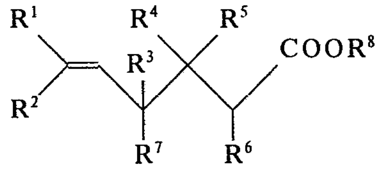

(IV)

dans laquelle

| | |
|---|---|
| $R^1$ et $R^2$ | sont identiques ou différents et représentent chacun un atome d'hydrogène ou un atome d'halogène, |
| $R^3$ à $R^6$ | sont identiques ou différents et représentent chacun un atome d'hydrogène ou un groupe alkyle éventuellement substitué, |
| $R^7$ | représente un atome d'halogène, |
| $R^8$ | représente un radical d'hydrocarbure éventuellement substitué, |

par réaction d'un composé de formule générale (V):

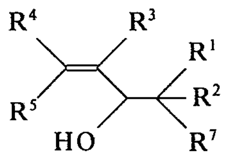

(V)

dans laquelle

| | |
|---|---|
| $R^1$ à $R^5$ | ont les significations indiquées ci-dessus, et |
| $R^7$ | représente un atome d'halogène, |

avec un orthoester de formule générale (VI):

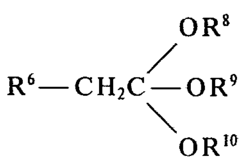

(VI)

dans laquelle

| | |
|---|---|
| $R^6$ | a la signification indiquée ci-dessus, et |
| $R^8$, $R^9$ et $R^{10}$ | sont identiques ou différents et représentent chacun un radical d'hydrocarbure éventuellement substitué, |

ou avec un cétène-acétal de formule générale (VII):

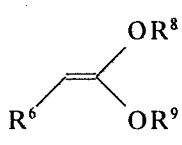

(VII)

dans laquelle

| | |
|---|---|
| $R^6$, $R^8$ et $R^9$ | ont les significations indiquées ci-dessus, |

éventuellement dans un solvant organique et éventuellement en présence d'un catalyseur acide, caractérisé en ce qu'on traite le mélange obtenu lors de cette réaction avec, au maximum, un équivalent molaire d'un agent d'halogénation et d'un alcool.